# EUROPEAN PATENT APPLICATION

(11) **EP 1 398 049 A1**
(43) Date of publication of application: **17.03.2004**
(21) Application number: 02388060.2
(22) Date of filing: 09.09.2002
(51) Int. Cl.: A61M 5/142, A61M 5/14, G05D 7/01, F04B 43/04, F15C 5/00, B81B 1/00, B01J 19/00, B01L 3/00

(54) **Flow restrictor**

(71) Applicant: NOVO NORDISK A/S, 2880 Bagsvaerd (DK)
(72) Inventor: Pedersen, Per Elgard, 4690 Haslev (DK)

(57) **Abstract**

The present invention relates to a combined flow restrictor suitable for the controlled transfer of fluid from a first compartment to a second compartment. More specifically, the invention relates to a safety arrangement reducing the risks associated with diminished flow resistance through the flow restrictor. In a first aspect the present invention provides a combined flow restrictor having a first flow restrictor of a first type, and a second flow restrictor of a second type arranged in series with the first flow restrictor. In further aspect the present invention provides a method of manufacturing a combined flow restrictor, comprising the steps of manufacturing a first flow restrictor in a first manufacturing process, manufacturing a second flow restrictor in a second manufacturing process independent of the first manufacturing process, and providing a combined flow restrictor device comprising the first and second flow restrictors arranged in series with each other. For both the device and the method, the combined flow restrictor may comprise further flow restrictors.

## Description

### FIELD OF THE INVENTION

The present invention relates to a flow restrictor suitable for the controlled transfer of fluid from a first compartment to a second compartment. More specifically, the invention relates to a safety arrangement reducing the risks associated with diminished flow resistance through the flow restrictor. Such flow restrictors are suitable for the application in fluid delivery devices of the bleeding hole type, such devices being suitable in particular for the in situ administration of a therapeutic drug preparation over a prolonged period of time, however, such devices may also be used in areas such as biochemistry, microbiology and chemical analysis.

### BACKGROUND OF THE INVENTION

In the disclosure of the present invention reference is mostly made to the treatment of diabetes by injection or infusion of insulin, however, this is only a preferred use of the present invention.

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes is straight forward substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides). However, the above-described deterioration is reflected in the fact that beta cell stimulators will eventually fail to stimulate the cell, and the patient has to be treated with insulin, either as mono therapy, or in combination with oral medication in order to improve glucose control.

Currently, there are two principal modes of daily insulin therapy, the first mode including syringes and insulin injection pens. These devices are simple to use and are relatively low in cost, but they require a needle stick at each injection, typically 3-4 times or more per day. The second mode is infusion pump therapy, which entails the purchase of a relatively expensive pump, for which reason the initial cost of the pump is a barrier to this type of therapy. Although more complex than syringes and pens, the pump offer the advantages of continuous infusion of insulin, precision in dosing and optionally programmable delivery profiles and user actuated bolus infusions in connections with meals.

Basically the infusion pump comprises means for allowing the contained insulin to be transferred to the body of the patient. These means may take any desirable form providing the desired function, but presently pump arrangements comprising a conveying arrangement connected to the reservoir (i.e. an outlet to be associated with needle infusion means) and including a pressure or suction generating device for feeding the liquid contained in the reservoir by pressure or suction application from the reservoir to the body are preferred for transferring the insulin contained in the reservoir to the patient. In this respect a number of different principles may be utilized, e.g. osmotic pumps as known from for example US patents 4,340,048 and 4,552,561, piston pumps as known from for example US patent 5,858,001, membrane pumps as known from for example US patent 6,280,148, flow restrictor pumps (also known as bleeding hole pumps) as known from for example US patents 2,605,765 and 5,957,895, and gas generating pumps as known from for example US patent 5,527,288, which all in the last decades have been proposed for use in durable (refillable) and/or disposable (prefilled) drug infusion systems. As some of these principles may not be considered to be pumps in the traditional sense, it may be more appropriate to generally describe these devices as delivery means for fluid, however, in the following description the traditional term pump will be used.

Of the above pump principles, the present invention specifically addresses the bleeding hole pump. Basically, this principle provides a means for establishing a flow of a fluid at a desired rate by applying a force to a liquid to thereby force the liquid through a flow restrictor, the flow rate being determined by the applied force, the flow resistance in the flow restrictor and the viscosity of the fluid. For the purpose of expelling a drug from a reservoir, two variants of this principle have been described.

In a first variant the drug to be infused is contained in a reservoir in fluid communication with an outlet through a flow restrictor. When the drug is pressurized by an actuating (driving) force it is forced through the flow restrictor at a rate determined by the applied force, the flow resistance in the flow restrictor and the viscosity of the drug, see for example US patent 5,957,895 which discloses an infusion device in which the driving force is provided by a drug reservoir formed between two Belleville springs, the flow restrictor being provided by the through-going channel of a capillary tube, or WO 02/15965 disclosing an infusion device in which the flow restrictor is in the form of a tortuous serpentine-formed channel established between two members. In the latter the flow resistance is selectable just as a bolus function is provided. Also US patent 5,993,414 discloses an infusion device utilizing a tortuous path flow restrictor.

In a second variant an infusion device comprises a first cavity containing a drive fluid, a flow restrictor comprising a flow channel, a second cavity in fluid communication with the first cavity through the flow channel, and a drug reservoir containing the drug to be infused, where the second cavity and the drug reservoir is arranged such that the volume of the drug reservoir diminishes when the volume of the second cavity increases. Further, drive means for expelling the drive fluid from the first to the second cavity through the flow restrictor is provided, whereby drug is expelled from the drug reservoir. The force-transmitting interface between the second cavity and the drug reservoir could be described as a secondary pump actuated by the drive means. As appears, the drug flow rate will be determined by the applied force, the flow resistance in the flow restrictor and the viscosity of the drive fluid. Advantages of the second variant are that the (delicate) drug does not have to be forced through the narrow flow restrictor and that a drive fluid having a high viscosity can be used thereby allowing a flow restrictor with a smaller flow resistance to be used which will normally be less expensive to manufacture. Examples of the second variant are disclosed in US patent 2,605,765 and German published patent application 25 52 446.

In the above referred infusion devices using the bleeding hole principle, it has been an object to provide a constant infusion rate which has been achieved using force generating means providing a near-constant force, e.g. different forms of springs. However, it is also possible to use the bleeding hole principle in combination with flow rate controlling means as known from the infusion device described in EP 1 177 802, this infusion device comprising processor controlled valve means which opens and closes the drug flow generated using a bleeding hole pump.

Although the bleeding hole principle thus has been proposed for a variety of drug infusion devices during the last five decades, it appears that the risks associated with this principle have until now not been properly identified. Basically, the infusion rate in a bleeding hole pump is determined by the applied force, the viscosity of the drive fluid and the flow resistance of the flow restrictor. Correspondingly, when it is a primary object to prevent drug overdosing it has to be assured that the applied force does not exceed the set limit, that the viscosity of the drive fluid is not below the set limit and that the flow resistance in the flow restrictor is not below the set limit. For the first two components it appears possible to assure with a relatively high certainty that the relevant parameters are within set limits (e.g. the risk that a given spring in a batch of springs is much stiffer than intended must be considered relatively low just as the viscosity of a given amount of drive fluid can be regarded as constant), just as it appears unlikely that these parameters will change substantially at a later stage (e.g. the viscosity may change with the temperature, however, not with a factor 2 or the like). In contrast, for the flow restrictor even small deviations during the manufacturing process may result in a dramatic change in flow resistance as the latter is influenced by the diameter of the flow channel in the fourth power. Even if it would be possible to measure and control every single flow restrictor before application in an infusion device, there would still be a risk that subsequent damage will result in a greatly reduced flow resistance and thus overdosing, e.g. a delicate structure in the flow restrictor may collapse or a bond may fail.

It thus appears that for a pump based on the bleeding hole principle the risk of overdosing is mostly associated with the potential run-away risk if the flow restrictor, e.g. due to manufacturing errors or damage, which would result in the pump rate being too high which ultimately may result in drug overdosing.

A solution to this problem could be the provision of a flow sensor arranged close to the drug outlet and cooperating with alarm means, however, the provision of such additional systems would add considerably to the manufacturing costs, and be a major obstacle to the implementation of this pump principle for a prefilled, disposable infusion device.

### DISCLOSURE OF THE INVENTION

Having regard to the above-identified problems, it is an object of the present invention to provide a flow restrictor suitable for use in a bleeding hole pump, which provides improved safety and can be manufactured in a cost-effective manner.

More specifically, the present invention is based on the realization that the risks associated with a flow restrictor can be diminished if the flow restrictor is provided as a composite unit comprising at least two independently manufactured flow restrictors arranged in series. By this arrangement the risk of substantial failure of the flow restructure is greatly diminished. For example, in case each of two flow restrictors provides 50% of the intended flow resistance, the (unlikely) total failure of one of the flow restrictors would still provide half the intended flow resistance and thereby "only" the double infusion rate.

Thus, in a first aspect the present invention provides a combined flow restrictor having a first flow restrictor of a first type, and a second flow restrictor of a second type arranged in series with the first flow restrictor. To exclude portions of a combined flow path which have only a neglectable flow resistance, e.g. the bores or conduits conducting fluid to and from the flow restrictor, it may be defined that each flow restrictor provides a "significant portion" of the combined flow resistance. For most practical purposes these structures are considered to posses a flow resistance of zero when determining the total flow resistance for a given flow path, e.g. between two cavities. More specifically, it may be defined that each of the first and second flow restrictors provides at least 5%, preferably at least 10%, and more preferably at least 25% of the combined flow resistance.

Each of the first and second flow restrictors may be selected from the group of flow restrictors comprising the types: capillary tube, micro opening, micro channel, micro bore, micro porous membrane and porous material.

Advantageously at least one further flow restrictor is arranged in series with the first and second flow restrictors, where each further flow restrictor may be of the first, second or a further type.

Preferably no single flow restrictor or type of flow restrictor provides more than approximately 50% of the combined flow resistance. For a combined flow restrictor comprising a plurality of flow restrictors or types of flow restrictors, preferably no single flow restrictor or type of flow restrictor provides more than approximately 25% of the combined flow resistance.

The individual flow restrictors may be arranged in individual members or one member may comprise two or more flow restrictors.

In the above described embodiments the flow restrictors have been characterized as being of different types, however, the same advantages can be achieved by providing at least two individual flow restrictors of the same type but manufactured independently of each other, e.g. using different manufacturing processes for manufacturing the same type of flow restrictor. The term "different manufacturing processes" includes manufacturing processes of the same type, e.g. a first flow restrictor hole may be manufactured by a first laser-drill set-up and a second flow restrictor hole may be manufactured by a second laser-drill set-up.

Thus, in a second aspect the present invention provides a method of manufacturing a combined flow restrictor, comprising the steps of manufacturing a first flow restrictor in a first manufacturing process, manufacturing a second flow restrictor in a second manufacturing process independent of the first manufacturing process, and providing a combined flow restrictor device comprising the first and second flow restrictors arranged in series with each other. Corresponding to the discussion above, it may be defined that each flow restrictor provides at least 5%, preferably at least 10%, and more preferably at least 25% of the combined flow resistance.

Advantageously, the method comprises the further steps of manufacturing at least one further flow restrictor using the first, the second or a further manufacturing process, and providing a combined flow restrictor device comprising the first and second flow restrictors and at least one further flow restrictor, the flow restrictors being arranged in series with each other providing a combined flow restrictor in which the flow restrictor(s) from one manufacturing process preferably provide(s) at least 5%, more preferably at least 10% and most preferably at least 25% of the flow resistance for the combined flow resistor.

In a third aspect the present invention provides a method of manufacturing a combined flow restrictor, comprising the steps of manufacturing a plurality of flow restrictors using a plurality of independent manufacturing processes, and providing a combined flow restrictor device comprising the plurality of flow restrictors arranged in series with each other.

In a fourth aspect the present invention provides a method of manufacturing a combined flow restrictor, comprising the steps of manufacturing in a first manufacturing process a first member comprising a first flow restrictor or group of first flow restrictors, manufacturing in a second manufacturing process a second member comprising a second flow restrictor or group of second flow restrictors, and providing a combined flow restrictor device comprising the flow restrictors of the first and second member, at least one first and one second flow restrictor being arranged in series with each other providing a combined flow restrictor, the flow restrictor(s) from one manufacturing process preferably provide(s) at least 5%, more preferably at least 10%, and most preferably at least 25% of the flow resistance for a combined flow resistor.

In preferred embodiment the method comprises the steps of manufacturing a plurality of members each comprising at least one flow restrictor, thereby providing a plurality of flow restrictors, wherein at least two independent manufacturing processes are used for the manufacture of the flow restrictors, and providing a combined flow restrictor device comprising the plurality of flow restrictors.

For a flow restrictor manufactured in accordance with the present invention, preferably the flow restrictor(s) manufactured from a single manufacturing process provides approximately 50% or less of the combined flow resistance. For a combined flow restrictor comprising a plurality of flow restrictors or types of flow restrictors, preferably no single flow restrictor or type of flow restrictor provides more than approximately 25% of the combined flow resistance.

In a fifth aspect the present invention provides a delivery device comprising a first variable volume cavity containing a drive fluid, a flow restrictor as described above, a second variable volume cavity in fluid communication with the first variable volume cavity through the flow channel, a variable volume drug reservoir having in a situation of use an outlet means, where the second variable volume cavity and the variable volume drug reservoir are arranged such that the volume of the drug reservoir diminishes when the volume of the second cavity increases. The delivery device further comprises drive means for expelling the drive fluid from the first to the second cavity through the flow restrictor such that drug is expelled from the drug reservoir through the outlet.

However, a delivery device of the above type may also be manufactured or offered to the user as a system in which individual components are combined with each other to provide an aggregate device. For example, it may be desirable to offer a system comprising a disposable, pre-filled drug unit, a durable drive-force providing unit and a disposable unit comprising the drive fluid and the flow restrictor. For such a system different flow restrictors providing different infusion rates in combination with a given drive-force could be offered.

The outlet means may be adapted to be brought in fluid communication with infusion means (e.g. a catheter tubing or transcutaneous access means such as an infusion needle, a flexible infusion cannula or a plurality of micro-penetrators) or may comprise these. In the latter case the fluid communication may be established just prior to use, before or after the drug delivery device has been arranged on the user.

Correspondingly, in a sixth aspect the present invention provides a fluid transmitting device comprising a first variable volume cavity containing a drive fluid, a flow restrictor as discussed and described above, a second variable volume cavity in fluid communication with the first variable volume cavity through the flow channel.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the preferred embodiments reference will be made to the use of insulin. Correspondingly, the term "infusion" is meant to encompass any method of parenteral delivery to a subject.

### BRIEF DESCRIPTION OF THE DRAWINGS

In the following the invention will be further described with references to the drawings, wherein
fig. 1A is an exploded perspective view of a first flow restrictor device in accordance with the invention,
fig. 1 B is an exploded perspective view of a second flow restrictor device in accordance with the invention,
fig. 2A shows a perspective view of an infusion device in an initial state,
fig. 2B shows a perspective view of the infusion device of fig. 2A in an actuated state,
fig. 3 shows a "horizontal" cross-sectional view of the infusion device of figs. 2,
fig. 4 shows a first "vertical" cross-sectional view of the infusion device of figs. 2,
fig. 5 shows a second "vertical" cross-sectional view of the infusion device of figs. 2,
fig. 6 shows in detail a flow restrictor, and
fig. 7 shows in detail a subcutaneous infusion needle.

### DESCRIPTION OF THE PREFERRED EMBODIMENTS

When in the following terms as "upper", "lower", "right" and "left" or similar relative expressions are used, these only refer to the appended figures and not to an actual situation of use. Further, the term "trace" is used to describe an "open" structure formed in a surface or a through-going in a member, e.g. a groove, whereas the term "channel" is used to describe a "closed" tubular structure which may have any cross-sectional configuration.

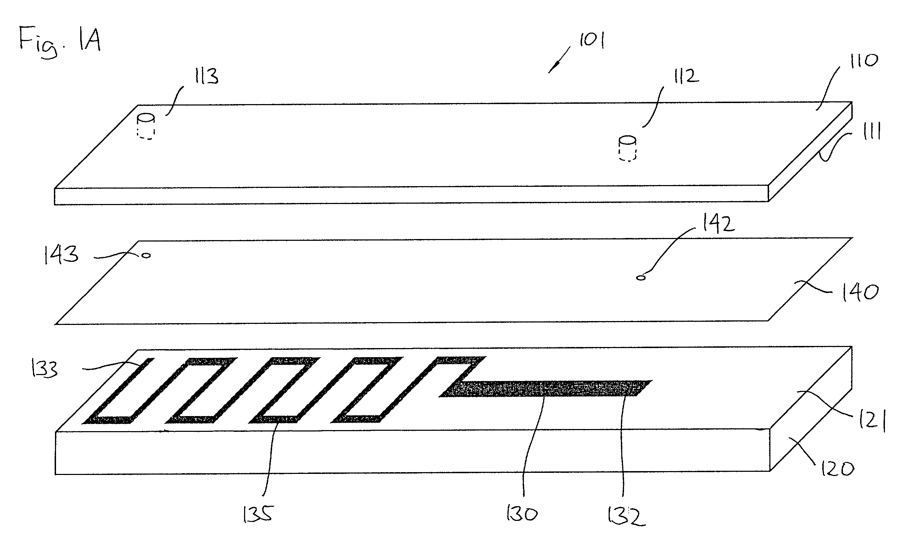

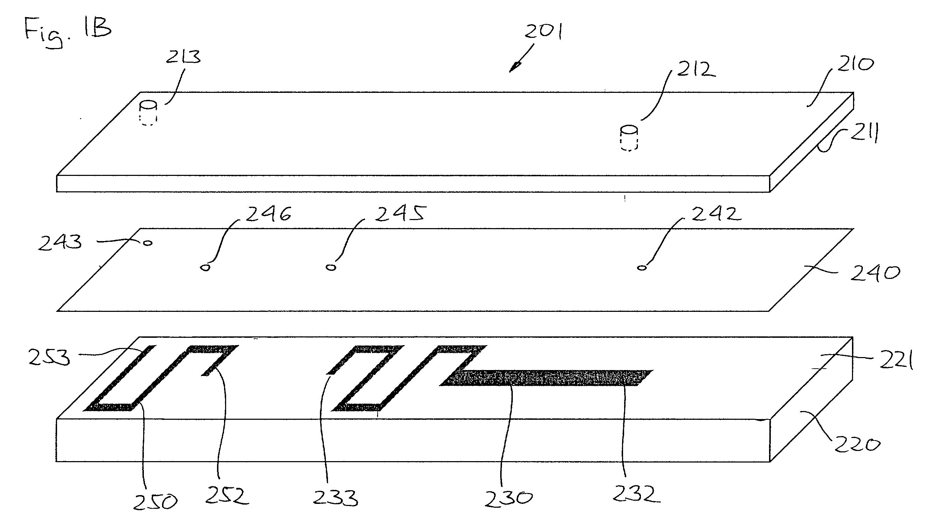

Fig. 1A shows a schematic representation of a first embodiment of the invention. Correspondingly, the configuration of the different structures as well as there relative dimensions are intended to serve illustrative purposes only.

More specifically, a flow restrictor device 101 comprises an upper member 110 with a generally planar lower surface 111 (cannot be seen in fig. 1A), a lower member 120 with a generally planar upper surface 121, and a generally planar intermediate member 140. The upper member comprises first and second through-going bores 112, 113 serving as inlet respective outlet for the flow restrictor.

In the upper surface is formed a flow trace 130 having an inlet end portion 132 and an outlet end portion 133 and a plurality of generally U-formed portions 135, the flow trace thereby forming a serpentine-like pattern. For illustrative purposes is the trace 130 shown as a relatively broad structure, however, for most applications the trace will have a width and depth in the micrometer range. The trace may be formed using any suitable technology, e.g. injection moulding, stamping, etching or by means of a laser. Although the trace is shown as a unitary structure, it may comprise two or more portions manufactured using two or more different manufacturing processes, e.g. different lasers.

The intermediate member 140 is in the form of a thin foil and comprises first and second openings 142, 143 serving as individual flow restrictors which are arranged to be in register with the bores 112, 113 in an assembled state of the device. The openings may be formed in the same or in two different processes. When a single manufacturing process is used, the two openings will belong to the same group of flow restrictors.

In an assembled state (not shown) the three members 110, 120, 140 are attached (e.g. bonded) to each other in a sandwich construction with their opposed surfaces in mating contact, whereby the flow trace will be "closed" to form a flow channel, the flow channel having an inlet end portion 132 serially in fluid communication with the first flow restrictor opening 142 and the inlet opening 112, and an outlet end portion 133 serially in fluid communication with the second flow restrictor opening 143 and the outlet opening 113. As indicated in the figure, the inlet and outlet openings have a considerably larger diameter whereby their flow resistance are neglectable compared to the "real" flow restrictors 142, 130, 143.

The flow resistance for the different flow restrictors may be chosen in accordance with the specific design of the combined flow restrictor device and the number of individual flow restrictors incorporated. For example, in case a first manufacturing process is used for the trace 130 and a second manufacturing process is used for the two flow restrictor openings 142, 143, then a distribution of 25% for each of the openings and 50% for the trace may be suitable.

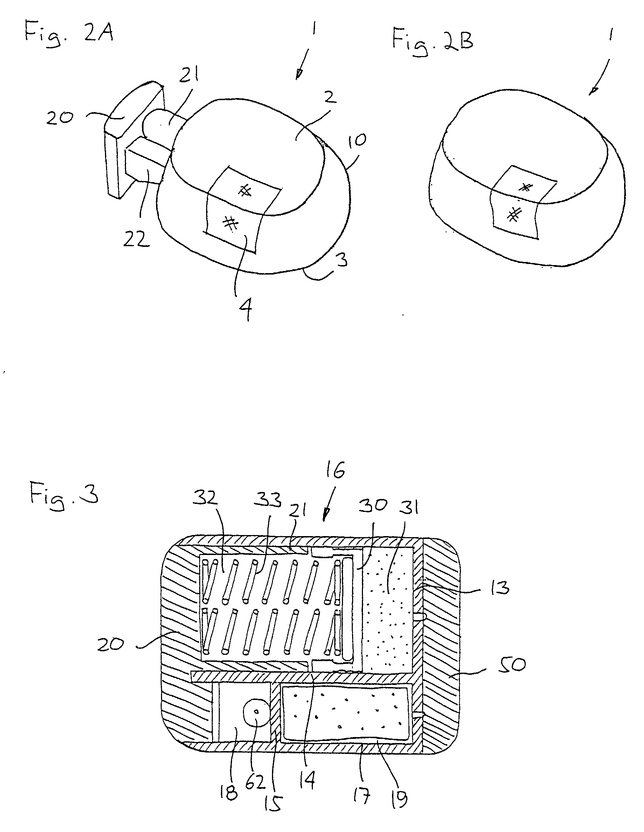

Fig. 1B shows a schematic representation of a second embodiment of the invention. As in the first embodiment the flow restrictor device 201 comprises an upper member 210 with a lower surface 211 and a lower member 220 with an upper surface 221 and an intermediate foil member 240 sandwiched between the two opposed surfaces, the three members being adapted to be bonded together to form a laminate structure.

In the upper surface 221 are formed first and second flow traces 230, 250 each having an inlet end portion 232, 252 and an outlet end portion 233, 253 and a plurality of generally U-formed portions 235, 255, the flow traces thereby forming serpentine-like patterns. In the lower surface 211 are formed a third flow trace having inlet and outlet end portions (not to be seen), the trace being arranged corresponding to the area between the first and second flow traces. The three flow traces may be manufactured using one, two or three different manufacturing processes thereby defining three, two or one group(s) of flow restrictors.

The intermediate member 240 member comprises first and second flow restrictors 242, 243 arranged to be in register with the bores 112, 113 in an assembled state of the device, as well as third and fourth flow restrictors 245, 246 arranged to be in register with end portions of the flow paths to thereby provide a serially connected flow path. The openings may be formed in the same or in different processes forming one or more group of flow restrictors.

In an assembled state (not shown) the three members 210, 220, 240 are attached (e.g. bonded) to each other in a sandwich construction with their opposed surfaces in mating contact, whereby each flow trace will be closed to form a corresponding flow channel. When properly arranged in register with each other, a single combined flow restrictor is formed through the first opening 242, the first flow channel 230, the third opening 245, the third flow channel, the fourth opening 246, the second flow channel 250 and the second opening 243.

When a flow channel is established by a combination of a flow trace and an opposed "closing" surface, the flow channel (and thus the flow restrictor) is considered formed in the member in which the flow trace is formed.

Fig. 2A shows a schematic representation of an embodiment of the invention. Correspondingly, the configuration of the different structures as well as there relative dimensions are intended to serve illustrative purposes only.

More specifically, fig. 2A shows an infusion device 1 comprising a housing 10 and a therefrom protruding actuation button 20. The housing comprises an upper surface 2 and a lower surface 3 (not to be seen) adapted to be arranged against a skin surface of a user. The upper surface is provided with a transparent window 4 allowing the user to view a drug reservoir arranged within the housing. In fig. 2B the infusion device has been arranged against the skin of a user and the actuation button has pressed into the housing by the user thereby actuating the infusion device as will be explained in detail below.

With reference to figs. 3-5 the general construction of the infusion device will be described. The housing comprises an upper wall 11, a lower planar base plate 12, side wall portions, an end wall 13 with an outer planar surface, and relative to the latter an opposed open end. Internally the housing comprises a first central wall 14 and a second oblique wall 15 in combination defining three compartments, a drive compartment 16, a reservoir compartment 17 and a needle compartment 18. The drive compartment forms a flat cylinder with an open proximal end and a substantially closed distal end. A piston 30 is slidingly arranged in the cylinder dividing the drive compartment in a distal fluid compartment 31 (corresponding to the above-described first cavity) filled with a viscous drive fluid (e.g. silicon oil), and a proximal spring compartment 32. The actuation button 20 comprises a skirt portion 21 slidingly received in the cylinder thereby closing the spring compartment. In the spring compartment are arranged two helical compression springs 33 acting on the piston, however, any compressible material or member providing a spring action or any other means providing or generating a force (e.g. gas generating means or a liquid/gas mixture) acting on the piston may be utilized. The actuation button further comprises a wedge portion 22 to be received in the needle compartment.

As best seen in fig. 5 the reservoir compartment comprises a flexible drug reservoir 40 with an insulin-containing drug formulation. The reservoir is preferably manufactured from a transparent material allowing the user to view and control the drug through the window 4. In the initial state, i.e. before any drug has been expelled from the infusion device, the reservoir has a configuration substantially corresponding to the configuration of the reservoir compartment, thereby forming a neglectable cavity 19 (or dead-space) between the two components. In case an air filled dead space is not acceptable, the space may be filled with a fluid (for illustrative purposes is the gap between the reservoir and the reservoir compartment relatively large). As appears, the dead-space represents the above-described second cavity in a substantially fully collapsed state. Inside the drug reservoir is arranged a U-formed membrane element 41 formed from a self-sealing material and comprising upper and lower membrane portions 42, 43. In the end portion 13 is formed an outlet opening 34 from the fluid compartment and an inlet opening 44 to the reservoir compartment.

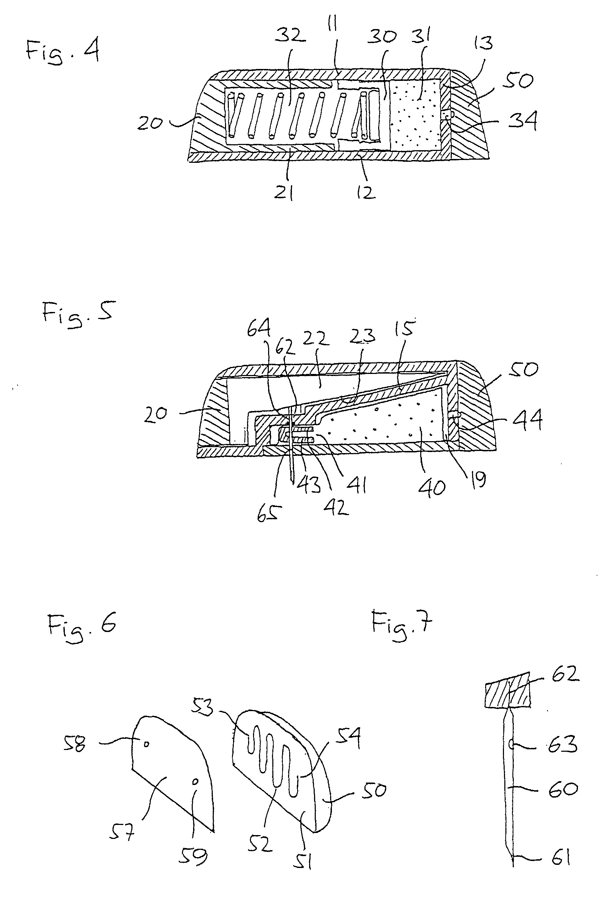

The infusion device further comprises an outer flow restrictor member 50 (see fig. 6) with a planer surface 51 in which a serpentine flow trace 52 is formed between proximal and distal end portions 53, 54, as well as an intermediate member 57 in which first and second flow restrictor openings 58, 59 are formed. The flow restrictor member 50 and the intermediate member 57 are bonded to the outer planar surface of the housing end portion in a sandwich arrangement with the proximal and distal end portions of the flow trace, respectively the first and second flow restrictor openings, in register with the outlet 34 respectively the inlet openings 44. In this way a combined flow restrictor channel is formed between the two openings. As appears, the resistance of the flow restrictor, the viscosity of the drive fluid and the force provided by the compressed springs will determine the rate at which the drive fluid will be forced through the flow restrictor to the reservoir compartment.

The infusion device further comprises a hollow subcutaneous infusion needle 60 as shown in fig. 7, comprising a distal pointed end 61 adapted to be introduced through a skin surface, a closed proximal end at which a needle wedge 62 is formed. In the body of the needle an opening 63 is formed in flow communication with interior of the needle. The proximal end of the needle is arranged in the needle compartment and with the needle body protruding through an opening 64 formed in the first wall 15 into the reservoir compartment and further into the reservoir. In the initial state (as supplied to the user and not shown in fig. 5) the needle penetrates the upper membrane portion 42 with the distal end 61 arranged between the upper and lower membrane portions 42, 43 inside the reservoir.

Next, with reference to figs. 4 and 5 actuation of the infusion device will be described. When the device has been positioned on a skin surface (preferably the lower surface comprises an adhesive coating) the user actuates the device by fully depressing the actuation button 20 until it locks in place in a recessed position (locking means arranged between the button and the housing is not shown in the figs.) whereby simultaneously the springs 33 are compressed and the wedge portion 22 is moved into the needle compartment. The wedge portion comprises a lower oblique surface 23 in sliding contact with the needle wedge 62 whereby the wedge portion forces the needle downwardly as it is pressed into housing. By this action the pointed distal needle end 61 penetrates the lower membrane portion 43 and is forced out through an opening 65 formed in the base portion. As the infusion device is attached to the skin surface of the user, the infusion needle is hereby introduced through the skin. When the needle is in its fully extended position, the needle opening 63 is positioned between the two membrane portions whereby a fluid communication is established from the drug reservoir to the user. At the same time the drive fluid starts to be expelled from the fluid compartment 31 and through the flow restrictor to the second cavity portion 19 of the reservoir compartment 17 where it gradually will compress the flexible reservoir and thereby force out the therein contained insulin-containing drug through the needle and into the user.

Initially air will be expelled from the needle just as air trapped in the flow restrictor and around the drug reservoir (if any) may result in an initial higher infusion rate, however, these effects will be neglectable.

In the shown embodiment the expelling means in form of springs 33 are "energized" during actuation of the device, however, to reduce the force needed to actuate the button 20 the spring means 33 may be pre-tensioned and the drive fluid 31 correspondingly pre-pressurized, whereby alone puncturing of the reservoir by the needle will actuate the expelling means and thereby start infusion.

In the above description of the preferred embodiments, the different structures providing the desired relations between the different components just as the means providing the described functionality for the different components (i.e. force generating means, flow restrictor, flexible reservoir etc.) have been described to a degree to which the concept of the present invention will be apparent to the skilled reader. The detailed construction and specification for the different structures are considered the object of a normal design procedure performed by the skilled person along the lines set out in the present specification.

## Claims

1. A combined flow restrictor comprising:
- a first flow restrictor (130) of a first type,
- a second flow restrictor (142) of a second type arranged in series with the first flow restrictor.

2. A combined flow restrictor as defined in claim 1, wherein each of the first and second flow restrictors is selected from the group of flow restrictors comprising the types capillary tube, micro opening and micro channel.

3. A combined flow restrictor as defined in claim 1 or 2, further comprising:
- at least one further flow restrictor (143) arranged in series with the first and second flow restrictors,
- each further flow restrictor being of the first, second or a further type.

4. A combined flow restrictor as defined in claim 3, wherein each of the first, second and further flow restrictors are selected from the group of flow restrictors comprising the types capillary tube, micro opening and micro channel, wherein the at least one further flow restrictor may be of the same type as for the first or second flow restrictor or of a further type.

5. A combined flow restrictor as defined in any of claims 1-4 comprising:
- a first member (120) comprising the first flow restrictor (130) of the first type,
- a second member (140) comprising the second flow restrictor (142) of the second type.

6. A combined flow restrictor as defined in claim 5, comprising:
- at least one further member (110), each further member comprising at least one further flow restrictor arranged in series with the first and second flow restrictors,
- the further flow restrictor(s) being of the first, second or a further type.

7. A combined flow restrictor as defined in any of the previous claims, wherein each flow restrictor, or each type of flow restrictor, provides at least 5%, preferably at least 10%, and most preferably at least 25% of the combined flow resistance.

8. A combined flow restrictor as defined in any of the previous claims, wherein no single type of flow restrictor provides more than approximately 50% of the combined flow resistance.

9. A delivery device (1) comprising:
- a first variable volume cavity (31) containing a drive fluid,
- a combined flow restrictor (50, 57) as defined in any of the previous claims,
- a second variable volume cavity (19) in fluid communication with the first variable volume cavity through the flow channel,
- a variable volume drug reservoir (40) having in a situation of use an outlet means,
- the second variable volume cavity and the variable volume drug reservoir being arranged such that the volume of the drug reservoir diminishes when the volume of the second cavity increases, and
- drive means (33) for expelling the drive fluid from the first to the second cavity through the flow restrictor, whereby drug is expelled from the drug reservoir through the outlet.

10. A fluid transmitting device comprising:
- a first variable volume cavity containing a drive fluid,
- a combined flow restrictor as defined in any of the previous claims, and
- a second variable volume cavity in fluid communication with the first variable volume cavity through the flow channel.

11. A method of manufacturing a combined flow restrictor, comprising the steps of:
- manufacturing a first flow restrictor (130) in a first manufacturing process,
- manufacturing a second flow restrictor (142) in a second manufacturing process independent of the first manufacturing process, and
- providing a combined flow restrictor device comprising the first and second flow restrictors arranged in series with each other.

12. A method of manufacturing a combined flow restrictor as defined in claim 11, comprising the further steps of:
- manufacturing at least one further flow restrictor (143) using the first, the second or a further manufacturing process, and
- providing a combined flow restrictor device comprising the first and second flow restrictors and at least one further flow restrictor, the flow restrictors being arranged in series with each other providing a combined flow restrictor.

13. A method of manufacturing a combined flow restrictor as defined in claim 11, comprising the steps of:
- manufacturing a plurality of flow restrictors (230, 242, 243, 245, 246, 250) using a plurality of independent manufacturing processes, and
- providing a combined flow restrictor device comprising the plurality of flow restrictors arranged in series with each other.

14. A method of manufacturing a combined flow restrictor as defined in claim 11, comprising the steps of:
- manufacturing in a first manufacturing process a first member (220) comprising a first flow restrictor or group of first flow restrictors (230, 250),
- manufacturing in a second manufacturing process a second member (240) comprising a second flow restrictor or group of second flow restrictors (242, 243, 245, 246), and
- providing a combined flow restrictor device comprising the flow restrictors of the first and second member arranged in series with each other, at least one first and one second flow restrictor being arranged in series with each other providing a combined flow restrictor.

15. A method of manufacturing a flow restrictor as defined in claim 14, comprising the steps of:
- manufacturing a plurality of members (110, 120, 140) each comprising at least one flow restrictor (230, 242, 243, 245, 246, 250), thereby providing a plurality of flow restrictors, wherein at least two independent manufacturing processes are used for the manufacture of the flow restrictors, and
- providing a combined flow restrictor device comprising the plurality of flow restrictors.

16. A method of manufacturing a combined flow restrictor as defined in any of claims 11-15, wherein each flow restrictor or group of flow restrictors manufactured from a single manufacturing process provides at least 10 %, preferably at least 25%, of the combined flow resistance.

17. A method of manufacturing a combined flow restrictor as defined in any of claims 11-16, wherein no single type of flow restrictor or group of flow restrictors manufactured from a single manufacturing process provides more than approximately 50% of the combined flow resistance.

18. A method of manufacturing a delivery device, the delivery device comprising:
- a first variable volume cavity (31) containing a drive fluid,
- a combined flow restrictor (50, 57),
- a second variable volume cavity (19) in fluid communication with the first variable volume cavity through the flow channel,
- a variable volume drug reservoir (40) having in a situation of use an outlet means,
- the second variable volume cavity and the variable volume drug reservoir being arranged such that the volume of the drug reservoir diminishes when the volume of the second cavity increases, and
- drive means (33) for expelling the drive fluid from the first to the second cavity through the flow restrictor, whereby drug is expelled from the drug reservoir through the outlet, the method comprising the steps of manufacturing a combined flow restrictor as defined in any of claims 11-17.

19. A method of manufacturing a fluid transmitting device, the fluid transmitting device comprising:
- a first variable volume cavity containing a drive fluid,
- a combined flow restrictor, and
- a second variable volume cavity in fluid communication with the first variable volume cavity through the flow channel, the method comprising the steps of manufacturing a combined flow restrictor as defined in any of claims 11-17.
